(19) Europäisches Patentamt
European Patent Office
Office européen des brevets

(11) **EP 2 337 493 B1**

(12) **EUROPÄISCHE PATENTSCHRIFT**

(45) Veröffentlichungstag und Bekanntmachung des Hinweises auf die Patenterteilung:
**21.09.2016 Patentblatt 2016/38**

(21) Anmeldenummer: **09777834.4**

(22) Anmeldetag: **12.08.2009**

(51) Int Cl.:
*A61B 5/022* (2006.01)  *A61B 5/024* (2006.01)
*A61B 5/00* (2006.01)  *A61B 5/021* (2006.01)
*A61B 5/11* (2006.01)

(86) Internationale Anmeldenummer:
**PCT/EP2009/005850**

(87) Internationale Veröffentlichungsnummer:
**WO 2010/017973 (18.02.2010 Gazette 2010/07)**

(54) **GERÄT ZUM KONTINUIERLICHEN MESSEN DES BLUTDRUCKS ZU ÜBERWACHUNGSZWECKEN**

EQUIPMENT FOR CONTINUALLY MEASURING THE BLOOD PRESSURE FOR MONITORING PURPOSES

APPAREIL DE MESURER LA PRESSION SANGUINE CONTINUELLEMENT POUR LE SENS DE SURVEILLANCE

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**

(30) Priorität: **15.08.2008 CH 12952008**

(43) Veröffentlichungstag der Anmeldung:
**29.06.2011 Patentblatt 2011/26**

(60) Teilanmeldung:
**13002184.3 / 2 620 100**

(73) Patentinhaber: **STBL Medical Research AG**
**8807 Freienbach (CH)**

(72) Erfinder:
• **SÜSSTRUNK, Heinz**
  **CH-8135 Langnau (CH)**
• **HIRT, Etienne**
  **CH-8005 Zürich (CH)**

(74) Vertreter: **Schaad, Balass, Menzl & Partner AG**
**Dufourstrasse 101**
**Postfach**
**8034 Zürich (CH)**

(56) Entgegenhaltungen:
WO-A-01/85024   WO-A-97/03606
WO-A-99/08591   WO-A-2004/004558
DE-A1-102005 059 435   US-A- 4 409 983
US-A- 5 494 043

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Die vorliegende Erfindung betrifft ein Gerät zum kontinuierlichen Messen des Blutdrucks zu Überwachungszwecken gemäss Anspruch 1. Das Gerät eignet sich insbesondere zur Langzeitmessung des Blutdrucks am gesunden wie am kranken Körper, damit ein gefährlich erhöhter oder erniedrigter Blutdruck frühzeitig erkannt werden kann.

Wenn das Herz schlägt, so entsteht mit jedem Schlag bzw. jeder Herzkontraktion eine Druckwelle, die sich vom Herz aus in den ganzen Körper ausbreitet. Der Körper besteht zum allergrössten Teil aus Wasser, sodass diese Ausbreitung nicht nur in den Arterien erfolgt, sondern sich auf alle Blutgefässe erstreckt, und Letztlich auf das ganze Körpergewebe. Mit jedem Herzschlag wird die Arterie elastisch erweitert und kontrahiert anschliessend wieder. Dabei wird die Blutsäule ab dem Herzen weitergeschoben, und ein Zurückfliessen wird durch das Schliessen der Herzklappen verhindert. Der Blutdruck ist am höchsten, wenn sich das Herz zusammen zieht (Dauer ca. 0,15 sekunden) und das Blut in die Arterien presst, die sich dadurch ausdehnen. Der dabei entstehende maximale Druck heisst systolischer Blutdruck. Der Blutdruck ist am niedrigsten, wenn das Herz wieder erschlafft (Dauer 0,7 Sekunden) und die Gefässe wieder ihren Normalzustand erreichen. Der dabei auftretende Druck heisst diastolischer Blutdruck. Genannt wird immer zuerst der systolische, dann der diastolische. 140/80 (gesprochen 140 zu 80) bedeutet also, dass der systolische Blutdruck den Wert 140, der diastolische den Wert 80 hat. Der Blutdruck ist aber nicht immer gleich, sondern schwankt während des Tages. Einflussgrössen sind beispielsweise Tageszeit, Stress, körperliche Belastung, Rauchen, Ausruhen und Entspannen.

Die gebräuchlichsten Methoden der nichtinvasiven Blutdruckmessung sind das sphygmomanometrische und das plethysmographische Messverfahren. Beide stellen Messungen des Blutdrucks mit Hilfe einer Druckmanschette gemäss dem Prinzip von Riva-Rocci dar. Es handelt sich um indirekte Messverfahren, bei denen die Extremwerte im Druckbereich des arteriellen Systems gemessen werden. Sie erlauben nur Gelegenheitsmessungen und keine kontinuierliche Erhebung der Druckverhältnisse. Die auskultatorische Messung mit einer Quecksilbersäule (Sphygmomanometer) gilt als historisch gewachsener "Goldstandard" der indirekten Blutdruckmessung (Riva-Rocci-Methode). Die anderen Messverfahren werden üblicherweise mit diesem Standard verglichen. Heutige Therapieverfahren bauen zumeist auf der auskultatorischen Messung auf.

Die oszillometrische Methode ist seit Anfang des 20. Jahrhunderts bekannt. Fast alle halbautomatischen und automatischen Geräte auf dem Markt messen oszillometrisch. Die Basis dieser Messmethode bilden die Druckschwankungen der Pulswelle (Oszillation). Diese Oszillation wird vom Druckwandler des Gerätes registriert und in Blutdruckwerte umgerechnet.

Heute verfügbare Geräte zur Blutdruckmessung am Handgelenk arbeiten mit dem oszillometrischen Messprinzip. Diese Geräte sind jedoch für den Einsatz in der Praxis und im Spital eher ungeeignet. Es sind initiale Vergleichsmessungen am Oberarm durchzuführen. In Europa müssen alle automatischen oder halbautomatischen Blutdruckmessgeräte nach einer Europäischen Norm validiert sein. Seit 1995 sieht diese CE-Zertifizierung (Europäische Norm) vor, dass nichinvasive Blutdruckmessgeräte das CE-Zeichen tragen dürfen, die nach der Europäischen Norm (EN-1060) für nichtinvasive Blutdruckmessgeräte validiert werden. Ziel der CE-Zertifizierung ist eine Harmonisierung der Gerätequalität in der Europäischen Union. Für elektronische Blutdruckmessgeräte sind fünf Prüfbereiche in der CE-Zertifizierung abgedeckt. Die CE-Zertifizierung verlangt, dass im Prüfverfahren für die Messgenauigkeit mindestens 85 Personen eingeschlossen werden. Die mittlere Abweichung - als Mass für die systematische Abweichung - mit einer auskultatorischen Messung am Oberarm darf höchstens 5mm Hg (Standardabweichung maximal 8mm Hg) betragen. Die Vergleichsmessungen erfolgen parallel am selben Arm, sequentiell oder parallel am andern Arm. Die CE-Zertifizierung garantiert ein technisch und apparatenmässig einwandfreies Funktionieren. Die Messvergleiche sollten aber nicht mit Patienten in einem hohen oder tiefen Blutdruck durchgeführt werden. Deshalb kann aufgrund dieser Validierung wenig über die Messgenauigkeit im klinischen Alltag ausgesagt werden. Das British Hypertension Society (BHS)-Protokoll zur Validierung von automatischen und halbautomatischen Blutdruckmessgeräten wurde 1990 eingeführt. Dieses Protokoll ist heute das am weitesten ausgefeilte Verfahren, das eine differenzierte Beurteilung der Messgenauigkeit zulässt. Die Beurteilung innerhalb der BHS-Validierung besteht aus dem Grading A, B, C und D, gemäss untenstehender Tabelle, welche die Differenz zwischen Standard Sphygmomanometer und Testapparat (mm Hg) aufzeigt:

| Gradierung | <5mm Hg | <10mm Hg | <15mm HG |
|---|---|---|---|
| A | 80% | 90% | 95% |
| B | 65% | 85% | 95% |
| C | 45% | 75% | 90% |
| D | Gradierung für C nicht erfüllt | | |

Die Prozentangaben beziehen sich auf die Messungen, die sich innerhalb der jeweils angegebenen Differenz befinden. Eine Gradierung B bedeutet, dass in 85% der Messungen Werte auftreten, die weniger als 10mm HG von der auskultatorischen Oberarmmessung abweichen. Ein Blutdruckmessgerät sollte mindestens eine Gesamt-Gradierung B/B (systolischer/diastolischer Blutdruck) aufweisen.

Nur klinisch validierte Geräte sollten für den professionellen Einsatz benutzt werden. Bisherige Geräte und Verfahren beruhen, mit Ausnahme der Pulswellen-Geschwindigkeitsmessung, meist auf dem Manschettenprinzip und erlauben damit nur eine Gelegenheitsmessung (Momentaufnahme) und keine kontinuierliche Erhebung der Druckverhältnisse.

Aus dem Dokument EP 1 341 436 ist eine Vorrichtung zur kontinuierlichen Überwachung des arteriellen Blutdrucks bekannt. Ihre Sensormittel sind an einem Ort benachbart zu einer Arterie auf der externen Oberfläche des Benutzerkörpers zu positionieren und mittels eines Gurts oder Bandes ist ein vorragender Abschnitt der Sensormittel derart stark gegen die Oberfläche zudrücken, dass eine wenigstens teilweise Okklusion der Arterie erfolgt. Dadurch wird der Blutfluss in der Arterie behindert.

Dokument WO 97/03606 offenbart ein nicht-invasives, nicht-intrusives, bequemes und tragbares Gerät zur Überwachung des arteriellen Blutdrucks eines Benutzers. Das kontinuierlich messende Blutdruck-ÜberwachungsGerät umfasst einen Wandler, der angeordnet ist um die Bewegung der Arterienwand, verursacht durch den Blutfluss durch eine Arterie des Benutzers, zu bestimmen und sein Aufbau dient der kontinuierlichen Messung der Ausgangssignale des Wandlers und der präzisen Umwandlung dieser kontinuierlich gemessenen Signale in systolische und diastolische Blutdruckwerte. Kalibrierdaten werden gesammelt, indem regelmäßig die Ausgangssignale des Wandlers mit gemessenen Ausgangssignalen eines separaten diagnostisch präzisen BlutdrucK-Messgerätes verglichen werden, wobei diese beiden Geräte bei der normalen Blutdruck-Überwachung nicht miteinander verbunden sind. Der Blutdruck wird aus den gemessenen Ausgangssignalen des Wandlers und den Kalibrierdaten berechnet.

Es ist eine Aufgabe der vorliegenden Erfindung, ein Gerät zu schaffen, um insbesondere am menschlichen Körper den Blutdruck zu Überwachungszwecken - ohne den Einsatz einer Druckmanschette - kontinuierlich zu messen.

Das Gerät soll dabei kompakt sein, ähnlich wie eine Armbanduhr, und bei fast allen körperliche Aktivitäten getragen werden könnten, gegebenenfalls ausser etwa beim Schwimmen oder Baden.

Diese Aufgabe wird mit einem Gerät mit den Merkmalen des Anspruchs 1 gelöst. Erfindungsgemässe wird an einer geeigneten Stelle des vorzugsweise menschlichen Körpers mittels eines Drucksensors der Druck gemessen. Der Drucksensor wird mittels eines Bandes - dieser Begriff umfasst beispielweise auch ein Band aus oder mit Kettengliedern - an der Stelle in sicherer, funktionsfähigen Anlage gehalten; die Anlage ist derart satt zu wählen, dass der Drucksensor, auch bei Bewegungen des Körpers, an der Stelle bleibt, jedoch nicht eine Arterie okkludiert oder teilokkludiert. Mit dem Drucksensor wird somit der Auflagedruck übergelagert von einer vom Blutdruck verursachten Druckschwankung gemessen.

Geeignete Stellen sind insbesondere an den Extremitäten vorhanden, beispielsweise am Unterarm, Oberarm oder Unterschenkel. Sie muss nicht über einer Arterie liegen, kann jedoch.

Um in der Verarbeitung des vom Drucksensor erzeugten Drucksignals den Auflagedruck kompensierten zu können, ist ein Anlagedrucksensor, der vorzugsweise in der Nähe des Drucksensors platziert ist, vorgesehen, wobei erfindungsgemäß der Auflagedrucksensor weniger über eine den Gehäuseboden des Geräts bildende Auflageplatte hervorsteht als der Drucksensor.

Der Anlagedrucksensor wird auch vom Band an die externe Oberfläche des Körpers in Anlage gehalten; vorzugsweise jedoch mit einem geringen Druck als der Drucksensor, derart, dass der Anlagedrucksensor den Druck, mit welchem er an der Oberfläche anliegt, jedoch mindestens annähernd ohne Überlagerung einer vom Blutdruck überlagerten Druckschwankung, misst.

Mittels eines Bandspannungssensors, der zusätzlich zum Anlagedrucksensor vorhanden ist, wird die Zugspannung des Bandes gemessen. Es versteht sich von selbst, dass sowohl der Bandspannungssensor als auch der Anlagedrucksensor ein entsprechendes Ausgangssignal generieren, das ein Mass für den Auflagedruck des Drucksensors darstellt.

Aufgrund unterschiedlicher Umstände kann sich der Umfang des Körpers beim Messort, beispielsweise der Umfang der betreffenden Extremität ändern. So ändert beispielsweise der Umfang bei Muskelanspannung, körperlicher Betätigung usw. Diese Änderung des Umfangs führt zu einer Änderung des Auflagedruckes des Drucksensors, was mittels des Anlagedrucksensors beziehungsweise Bandspannungssensors bei der Verarbeitung des Drucksignals berücksichtigt werden kann.

Bevorzugte Ausführungsformen der Erfindung sind in den abhängigen Ansprüchen angegeben.

Die Erfindung wird anhand der Zeichnung dargestellten Ausführungsformen näher erläutert. Es zeigen rein schematisch:

Fig. 1     eine perspektivische schematische Darstellung eines Gerätes zum kontinuierlichen Blutdruck-Messen für Überwachungszwecke;

Fig. 2    eine erstes Schema der Messanordnung und ihrer elektronischen Schaltung;

Fig. 3    Messergebnisse von vier Drucksensoren in Millivolt in einer Werte-Tabelle, sowie die Werte eines Beschleunigungssensors und die Temperaturwerte für die Haut;

Fig. 4    die Messergebnisse der vier Drucksensoren in Millivolt, aufgetragen gegen die Zeit;

Fig. 5    die Messwerte für den systolischen Druck graphisch dargestellt, in Korrelation zu den Ergebnissen einer oszillometrischen Messung des Blutdruckes;

Fig. 6    die Messwerte für den diastolischen Druck graphisch dargestellt, in Korrelation zu den Ergebnissen einer oszillometrischen Messung des Blutdruckes;

Fig. 7    einen Vergleich der Messwerte für den systolischen und den diastolischen Druck graphisch dargestellt, in Korrelation zu den Ergebnissen einer oszillometrischen Messung des Blutdruckes;

Fig. 8    in perspektivischer Darstellung eine weitere Ausführungsform das erfindungsgemässe Gerät mit einem in ein Armband integrierten Bandspannungssensor;

Fig. 9    eine Ansicht auf den Boden des Gehäuses des Geräts mit einer möglichen Anordnung von vier Sensoren;

Fig. 10   eine Aussicht auf den Boden des Gehäuses des Geräts mit einer möglichen weiteren Anordnung von drei Sensoren;

Fig. 11   einen Längsschnitt durch das Gehäuse mit u.a. einem Drucksensor und einem Anlagedrucksensor;

Fig. 12   in Seitenansicht ein als Kette ausgebildetes Band mit Verschluss;

Fig. 13   in Draufsicht die Kette mit Verschluss gemäss Fig. 12;

Fig. 14   einen Schnitt durch das Gehäuse mit einem Bandspannungssensor;

Fig. 15   in gleicher Darstellung wie Fig. 14 eine Ausführungsform mit einem unterschiedlichen Bandspannungssensor;

Fig. 16   ein zweites Schema der Messanordnung und ihrer elektronischen Schaltung;

Fig. 17   einen Überblick über die Anlaufschritte vom Start, nach dem Anlegen des Gerätes, bis zum Ende in Form eines Flussdiagramms;

Fig. 18   ebenfalls in Form eines Flussdiagramms Schritte der "Korrektur Armbandspannung Routine" der Fig. 17;

Fig. 19   ein Beispiel einer mittels der "Korrektur Armbandspannung Routine" ermittelten Korrekturfunktion;

Fig. 20   in Form eines Flussdiagramms Schritte der Routine "Ermittlung Umrechnungsfaktoren auf mm Hg" der Fig. 17; und

Fig. 21   ein Beispiel einer mittels der Routine gemäss Fig. 20 ermittelten Korrekturfunktion.

Mit dem Puls bzw. der von ihm erzeugten Druckwelle werden rote Blutkörperchen sowie wenige kleine Trombozythen in den Körper transportiert. Das Blut eines erwachsenen Menschen enthält ca. 5 Mio. rote Blutkörperchen und ca. 6000 weisse Blutkörperchen. Besonders die Anzahl der roten Blutkörperchen verhält sich linear zum Blutvolumen. Die Dichte der roten Blutkörperchen hat aber einen Einfluss auf die Absorption von Infrarotstrahlen, die etwa 1mm in die Haut eindringen, und deshalb lässt sich die Absorption bzw. Reflexion von in die Haut eindringender Infrarotstrahlung mit einem Infrarotsender/Detektor messen. Damit aber ist es möglich, den Puls über eine Erfassung der Infrarotabsorption bzw. Reflexion zu detektieren. Die Pulskurve kann daher in Form einer Reihe von elektrischen Signalen als Kurve dargestellt werden. Es ergibt sich eine sinusartige Kurvenform. Diese Tatsache wird für die verfahrensgemässe Ermittlung des Blutdruckes mitverwendet. Zum andern geht das Messverfahren von der Tatsache aus, dass der Körper zur Hauptsache aus inkompressiblem Wasser besteht, also in seinem Innern sozusagen eine wässrige Lösung bildet. Eine Druckwelle breitet sich daher im Körpergewebe praktisch ungedämpft aus. Dabei bezeichnet der transmurale Druck ein Partialdruck ist, und welcher dem Blutdruck entspricht.

Eine weitere gegebenenfalls zu berücksichtigende Einflussgrösse ist der Luftdruck, welcher von aussen auf den Körper einwirkt. Schliesslich kann eine Messung auch von der Beschleunigung der Mess-Stelle beeinflusst werden, die daher optional auch erfasst werden kann, um sie im Messergebnis zu kompensieren.

Das Gerät und das Verfahren zur kontinuierlichen Blutdruckmessung basieren auf der Messung der Interpretation oben erwähnter Werte. Das sei vorerst an einer beispielsweisen Versuchsanordnung erklärt. Er wurden vier Drucksensoren auf einer Auflageplatte mit der Hautoberfläche in Auflageberührung gebracht, und zwar am Unterarm in der Nähe des Handgelenks, gegenüber der Stelle, an welcher eine Armbanduhr getragen wird und wo man üblicherweise den Puls abgreift. Es sei an dieser Stelle erwähnt, dass weitere Versuche gezeigt haben, dass die Drucksensoren bzw. der Drucksensor auch an anderen geeigneten Stellen angebracht werden können, beispielsweise dort wo üblicherweise die Armbanduhr getragen wird, am Oberarm oder am Bein u.s.w.

Mit den genannten Drucksensoren wurde der Druck auf der Hautoberfläche gemessen, welcher den transmuralen Druck als Partialdruck enthält. Gesondert davon wurde an dieser Mess-Stelle der Puls ermittelt, indem mit einem Infrostsender Infrotstrahlung in das Gewebe gesandt wurde und die Reflexion detektiert wurde. Es konnte gezeigt werden, dass die Druckwellen des Pulses mit den Druckwerten, die von den vier Drucksensoren gemessen wurden, korrelierten. Man konnte daher stets anhand der Pulskurve auf der Druckwertkurve lokalisieren, wo der diastolische und wo der systolische Druck abgebildet wird. Dieses Bild erlaubte aber bloss eine qualitative Interpretation. Noch war der Blutdruck ein in den gemessenen Druckwerten enthaltener, überlagerter Wert. Zur Überprüfung der absoluten Werte konnte freilich nicht am gleichen Arm des Probanden auf konventionelle Art gemäss Riva Rocci mit einer Druckmanschette am linken Oberarm der Blutdruck bestimmt werden. Vielmehr wurden die gemessenen Daten mit jenen einer oszillometrischen Messmethode verglichen.

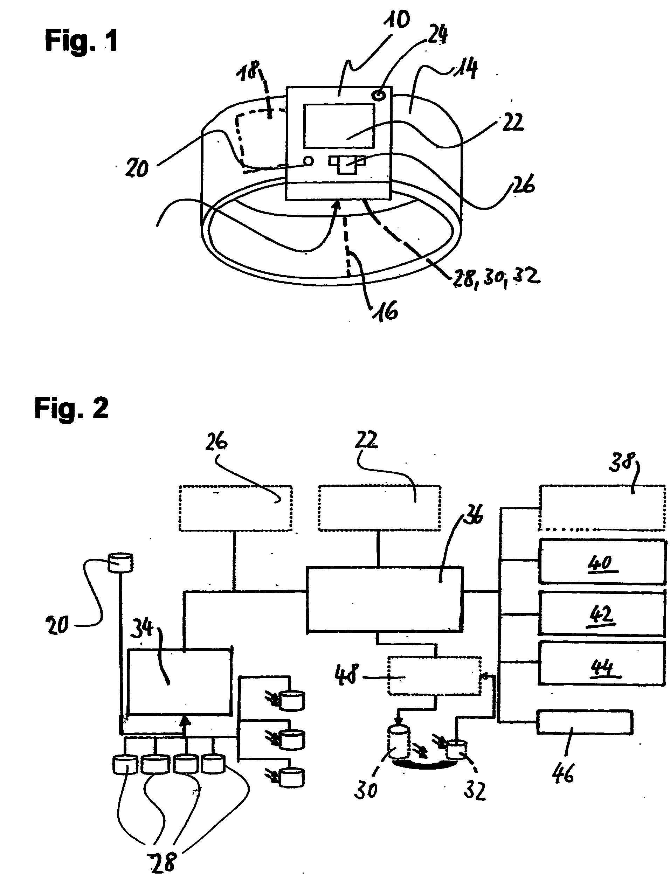

In Fig. 1 ist ein Gerät darstellt, das wie eine Armbanduhr getragen werden kann. Eine Auflageplatte 10, diese bildet hier ein Gehäuse 12, ist in ein Armband 14 integriert, welches mit einem nicht näher dargestellten Verschluss 16 ausgerüstet ist, und die Auflageplatte 10 kommt beim Tragen beispielsweise auf der Stelle des Unterarms zu liegen, welcher der normalerweise getragenen Armbanduhr gegenüberliegt und wo man üblicherweise den Puls abgreift; wie weiter oben erwähnt sind jedoch andere Stellen auch möglich.

Die Auflageplatte 10 soll gleichmässig und mit möglichst gelichbleibenden Anpressdruck an dieser Stelle am Unterarm anliegen. Das Armband 14 ist mit einem Dehnungssensor 18 ausgestattet, welcher ein Mass für die Bandspannung erfasst. Wenn das Armband 14 infolge einer temperaturbedingten oder infolge körperlicher Aktivität ausgelösten Schwellung des Armes gedehnt wird, so wird das vom Dehnungssensor 18 registriert und kann in der Messung kompensiert werden. Auf der Oberseite oder seitlich an der Auflageplatte ist ein Drucksensor 20 (Luftdrucksensor) zur Ermittlung des aktuellen Atmosphärendruckes vorhanden. Auf der Oberseite der Auflageplatte 10 weist diese einen Display 22 zur Anzeige des gemessenen diastolischen und systolischen Blutdruckes auf, sowie eine optische Warnleuchte 24. Ausserdem sind die Eingabetasten 26 abgeordnet.

Auf der Unterseite der Auflageplatte 10 und somit hier nicht einsehbar sind mehrere Drucksensoren 28 angeordnet, sodass sie mit ihrer sensitiven Seite gegen unten, das heisst gegen die Haut des Trägers, gerichtet sind. Ebenfalls auf der Unterseite ist ein Infrarot-Sensor 30 integriert, mit zugehörigem Detektor 32, sodass Infrarotlicht in die Haut des Trägers abgebar ist, und die Reflexion detektierbar ist. Im innern der Auflageplatte 10 ist eine integrierte Schaltung untergebracht, die im Folgenden näher beschrieben wird.

In Figur 2 ist ein Schema der Messanordnung und dieser elektronischen Schaltung gezeigt. Links unten sieht man die vier Drucksensoren 28 mit einer Sensitivität von 0 bis 1,5 bar. Sie enthalten beispielsweise je einen Piezokristall, sodass bei Druckbeaufschlagung eine Spannung abgegeben wird, die linear mit dem Druckwert verläuft. Ein weiterer Drucksensor, der Luftdrucksensor 20, misst den gerade herrschenden Atmosphärendruck. Die von diesen Drucksensoren 28 und dem Luftdrucksensor 20 abgegebenen Signale in Form von Spannungen gehen dann an einen AD-Converter 34 und von dort weiter an einen Mikroprozessor 36. Der Mikroprozessor 36 lässt sich über die Eingabetasten 26 bedienen, und seine Output-Werte können auf einem Display 22 zur Anzeige gebracht werden. Weiter ist der Mikroprozessor 36 mit einem RF-Interface 38 verbunden, sowie einem USB-Interface 40 zum Auslesen der Daten auf einen externen Computer. Der Mikroprozessor 36 ist ausserdem mit einem Speicher 42 verbunden, sowie mit einer Stromversorgung 44 (Batterie und Netzanschluss). Ausserdem ist ein Beschleunigungssensor 46 angeschlossen, dessen elektrische Signale an den Mikroprozessor 36 geliefert werden. Unterhalb des Mikroprozessors 36 ist der Infrarot-Sender 30 sowie der zugehörige Infrarot-Detektor 32 für die Messung der Pulse dargestellt. Mit 48 ist ein Verstärker zur Ansteuerung des Infrarot-Senders 30 und ein DA-Wandler bezeichnet.

An 15 Patienten, nämlich 5 Frauen und 10 Männern im Alter zwischen 27 und 59 Jahren wurde der Blutdruck mittels der vorliegenden Einrichtung und des vorliegenden Verfahrens gemessen, das heisst mit den vier Drucksensoren 28 wie in Figur 1 gezeigt, und diese wurden 5cm weg vom linken Handgelenk platziert bzw. mittels der Auflageplatte 10 angelegt, gegenüber der Stelle wo man die Armanduhr trägt, und die gewonnenen Werte wurde verglichen mit den Werten einer gleichzeitig vorgenommenen oszillometrischen Messung am rechten Oberarm, vorgenommen mit einem Gerät Acutron Mindray SV 800. Die Messungen wurden dabei in entspannter Sitzposition durchgeführt, einmal in völlig entspanntem, ausgeruhtem Zustand des Probanden, und einmal nach leichter körperlicher Anstrengung. Dabei wurden die Einzelmessungen über je 30 Sekunden durchgeführt, und zwar einmal in entspannter Sitzposition, einmal bei zugehaltener Nase und Mund und Einpressens von Atemluft in dieselben (Valsalva-Methode), einmal bei gleichzeitigem Fingertrommeln und einmal bei isometrischer Anspannung der Unterarm-Muskulatur, und schliesslich einmal bei fortwährender Pumpebewegung mit den Fingern (Hand zu Faust ballen und nach 30 Sekunden wieder lösen). Die Messergebnisse mit den Drucksensoren 28 (die Druckersignale) lagen zunächst in Millivolt vor. Weil keine Manschette zum Einsatz kam, ist der vorgenommene Vergleich nicht kompatibel mit den Forderungen der British Hypertension Sociey BHS oder auch der American Association for the Improvement of Scientific Apparatus AASI.

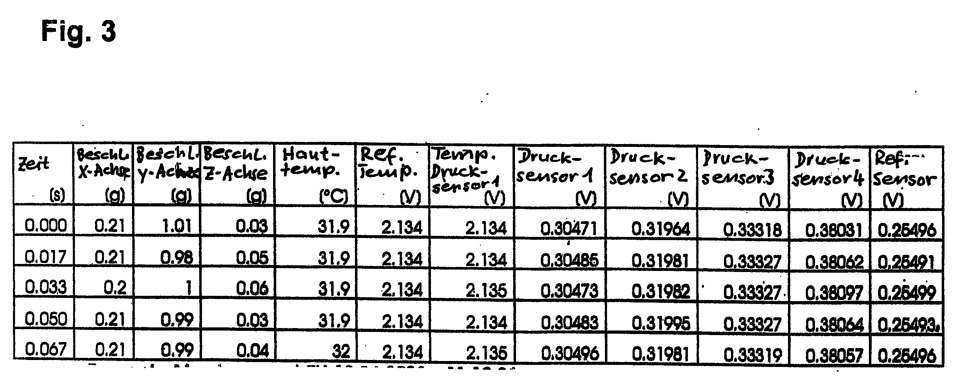

Die Figur 3 zeigt die Messergebnisses der vier Drucksensoren 28 in Millivolt anhand einer Wertetabelle, erfasst über eine Periode von 67 Sekunden. Ausserdem enthält diese Tabelle die Werte des zusätzlichen Beschleunigungssensors 46, sowie die gemessene Hauttemperatur und einen Referenzwert für die Sensortemperatur. In der hintersten Spalte ist der Druckwert des zusätzlichen Luftdrucksensors 20 angegeben. Insgesamt wurden 225 Messungen mit beiden Methoden durchgeführt und verglichen, also mit der konventionellen oszillometrischen wie auch der neuen, Drucksensor-basierten Methode. 108 dieser Vergleich wurden genauer untersucht. Es ist klar, dass weitere Messungen nötig sind, mit Personen verschiedenen Geschlechts, Alters, Grösse, Gewicht, initialem Blutdruckwerten, Armlängern etc.

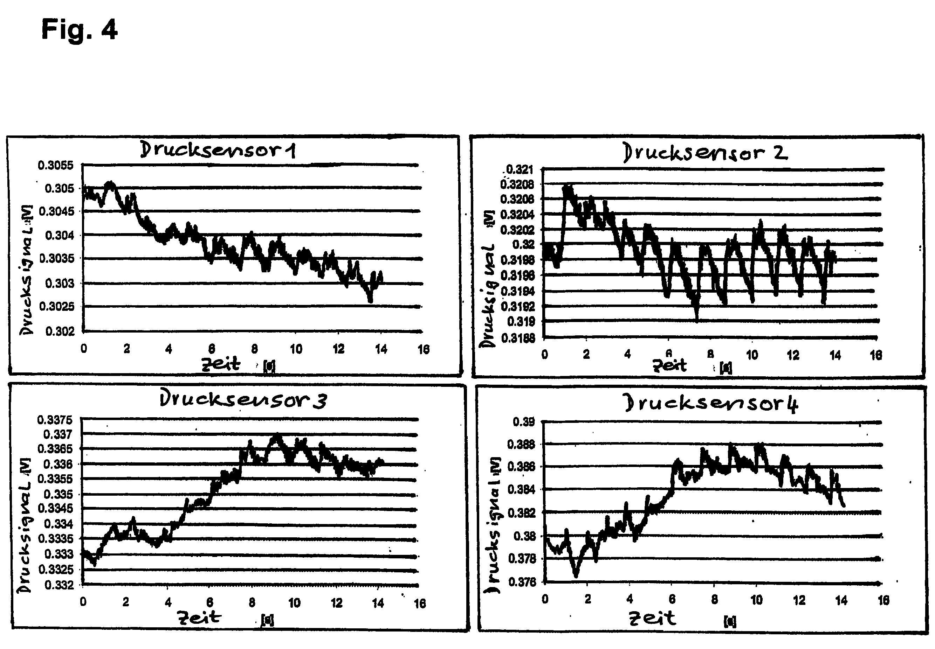

Die Figur 4 zeigt die Messergebnisses der vier Drucksensoren in Millivolt, aufgetragen gegen die Zeit. Man erkennt daran, dass jeder Wert getreulich zwischen lokalen Maxima und Minima schwankt, zwischen ca. 0.319mV und ca. 0.388 mV, und dass diese Peaks der vier Graphen miteinander zeitlich korrelieren. Von diesen vier Blutdruckkurven wurde jene mit den zweitniedrigsten Absolutwerten (Drucksensor 2) um den diastolischen Blutdruck und den dazugehörigen höheren systolischen Werten ausgewählt, und jene Kurve mit den höchsten Absolutwerten um den diastolischen Blutdruck (Drucksensor 4) und dem dazugehörigen höheren systolischen Blutdruck. Die Werte lassen sich ausmitteln und man erhält eine Schwankungskurve für den Druckwert, welcher den transmuralen Druck enthält.

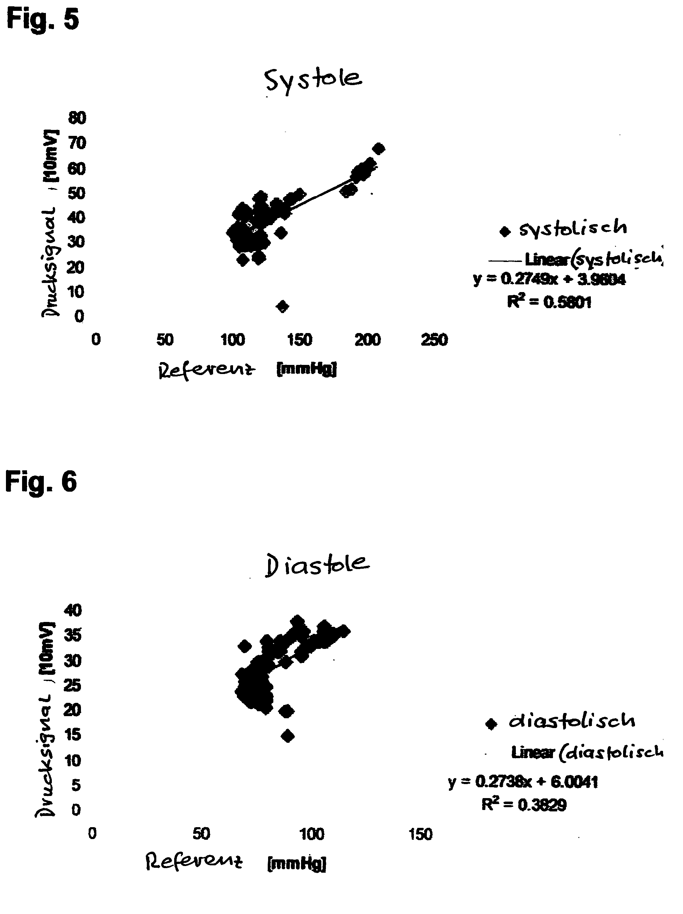

Die oszillometrisch gemessenen Blutdruckwerte sowie die Drucksensorwerte in Millivolt wurden für jede 30-Sekunden-Periode verglichen und in Korrelation zueinander gebracht. In Figur 5 ist aufgezeigt, wie diese gemittelten Messwerte für den systolischen Druck graphisch dargestellt mit den Ergebnissen einer oszillometrischen Messung des Blutdruckes zwischen 97 und 214mm Hg korrelieren. Und Figur 6 zeigt die Korrelation für den diastolischen Blutdruck zwischen 96 und 107mm Hg. Für den unteren, diastolischen, wie auch für den oberen, systolischen Blutdruckwert liess sich also eine lineare Funktion ermitteln. Dabei konnten die zu erwartenden Unterschiede in Bezug auf die Messungen der durch entspannte und angespannte Muskeln verursachten Grundspannung festgestellt werden.

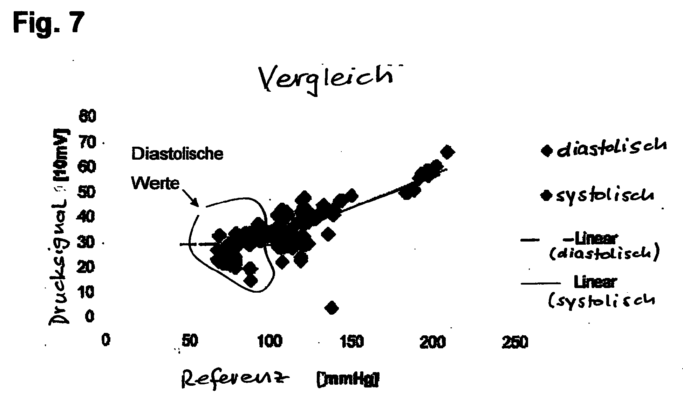

Dasselbe trifft auch zu für den Vergleich der beiden Blutdruckwerte wie in Figur 7 gezeigt. Hier sind die Messwerte für den systolischen und den diastolischen Druck graphisch dargestellt, in Korrelation zu den Ergebnissen einer oszillometrischen Messung des Blutdruckes. Es können drei verschiedene Ansammlungen von Messpunkten identifiziert werden. Eine im Bereich eines erhöhten Blutdruckes um bis zu 214mm Hg. Eine zweite Ansammlung von Messpunkte liegt im Bereich von ca. 145mm Hg, und eine dritte um 60 bis 120mm Hg. Die Linearität zwischen den Werten, die von den Drucksensoren 28 gewonnen wurden und jenen aus den oszillometrischen Werten bleibt unverändert und die Schwankungen der Messwerte liegen in einem schmalen Band.

Die Beziehung zwischen der als Referenz benützen oszillometrischen Methode und der Drucksensor-basierten Methode erscheint sehr präzise zu sein, mit einer deutlichen Linearität der Beziehung. Die Drucksensor-basierten Methode ist vielversprechend mindestens für Messungen am ruhenden oder nur leicht körperlich aktiven Probanden.

Bemerkenswert ist, dass kein qualitativer Unterschied zwischen den Vergleich beim systolischen und diastolischen Blutdruck festgestellt wurde. Die Ermittlung des diastolischen Blutdruckes, welcher generell schwieriger zu messen ist, erweist sich daher mit dieser Drucksensor-Methode als nicht anspruchsvoller als die Messung des systolischen Blutdruckes. Der Puls wie auch die Blutdruckkurve mit ihrem typischen Anstieg und der sogenannten C-Welle im Abfall sind deutlich identifizierbar. Das kann auch Rückschlüsse auf den Zustand der Blutgefässe erlauben. Während herkömmlich oszillometrische Geräte eine 30-Sekunden Periode für eine Messung der Blutdruckes benötigen, ist im diesem Drucksensor-Gerät und Verfahren eine Messung von Herzschlag zu Herzschlag möglich, das heisst eine kontinuierliche Messung und somit Überwachung rund um die Uhr. Sobald gewisse einstellbare Werte überschritten werden, kann ein akustisches oder optisches Signal am Gerät, oder via die Schnittstellen ein externer automatischer Alarm ausgelöst werden.

Unter Berücksichtigung der Werte des Beschleunigungssensors 46 können die Messwerte auch für einen Probanden in Bewegung kompensiert werden, also wenn der Proband etwa geht und seine Arme schwingt oder wenn er sitzt und seine Arme schwingt oder wenn er sitzt und seine Arme bewegt, etwa bei normaler Bürotätigkeit. Es versteht sich, dass die gemessenen Spannungswerte in jedem Einzelfall am Träger eines derartigen Gerätes justiert und regelmässig überprüft werden müssen. Aber durch das Tragen eines Gerätes, das auf dieser Basis funktioniert, kann der Blutdruck soweit laufend gemessen werden, dass eine überraschender Ausschlag insbesondere nach oben, aber auch nach unten feststellbar wird.

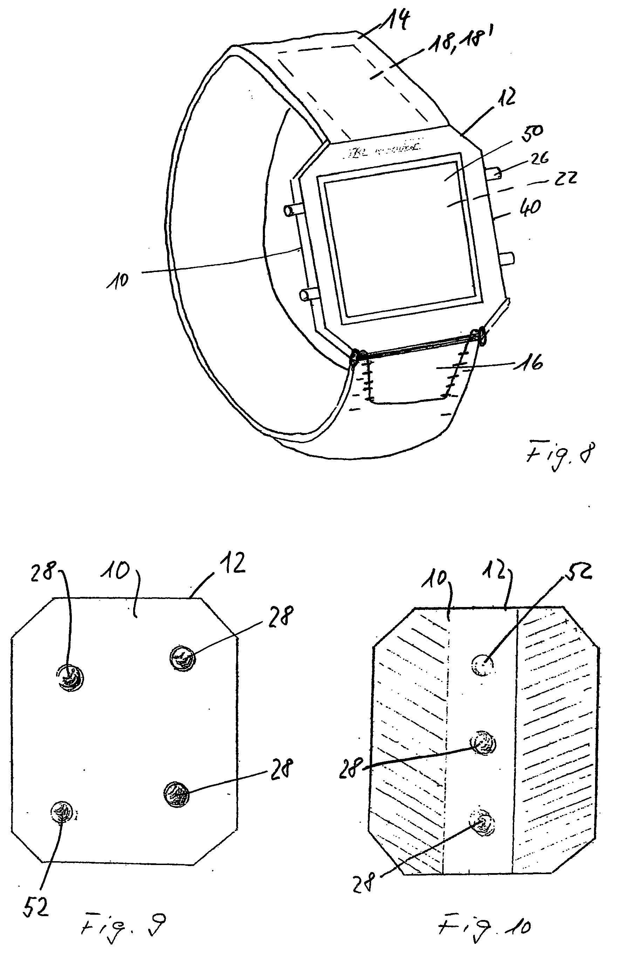

Fig. 8 zeigt eine weitere Ausführungsform des erfindungsgemässen Geräts mit dem Gehäuse 12 und dem an diesem in der Art einer Armbanduhr angelehnten Armband 14. Das Gehäuse 12 weist auf der Unterseite die Auflageplatte 10 und auf der Oberseite ein Abdeckglas 50 auf, unterhalb welchem sich das Display 22 befindet. Vier Eingabetasten 26 sind mantelseitig des Gehäuses 12 angeordnet. Weiter befindet sich mantelseitig der Stecker des USB-Interfaces 40.

Im Armband 14 befindet sich ein Bandspannungssensor 18' in Form des Dehnungssensors 18. Mittels des Verschlusses 16, lässt sich das Gerät an der geeigneten Stelle der Extremität ortsfest anbringen. In bevorzugterweise ist der Verschluss 16 als Stufenverschluss augebildet, um die Spannung, mit welchem das Armband 14 bzw. verallgemeinert das Band, stufenweise fester angezogen und stufenweise loser angezogen werden kann.

Die übrigen im bzw. am Gehäuse 12 angeordneten Elemente sind weiter unten im Zusammenhang mit der Beschreibung der Fig. 9 -16 erwähnt.

Fig. 9 zeigt in Untersicht das Gehäuse 12, somit die Unterseite der den Boden des Gehäuses 12 bildenden Auflageplatte 10. Dieser Auflageplatte 10 sind drei Drucksensoren 28 und ein Anlagedrucksensor 52 zugeordnet. Dieser Anlagedrucksensor 52 kann anstelle des Bandspannungssensors 18' vorhanden sein. Ist jedoch ein Bandspannungssensor 18' vorhanden, kann anstelle des Anlagedrucksensors 52 ein vierter Drucksensor 28 vorgesehen werden.

Im in der Fig. 9 gezeigten Ausführungsbeispiel sind die drei Drucksensoren 18 und der Anlagedrucksensor 52 annähernd in den Ecken eines Rhombus angeordnet und in einem Abstand zum Rand der Auflageplatte 10.

Fig. 10 zeigt in gleicher Darstellung wie Fig. 9 eine lineare Anordnung von zwei Drucksensoren 28 und dem Anlagedrucksensor 52 bzw. drei Drucksensoren 28, falls ein Bandspannungssensor 18' vorhanden ist. Die drei Sensoren befinden sich in einer Reihe entlang einer Geraden, welche sich in Längsrichtung des Armbands 14 und mittig der Auflageplatte 10 erstreckt.

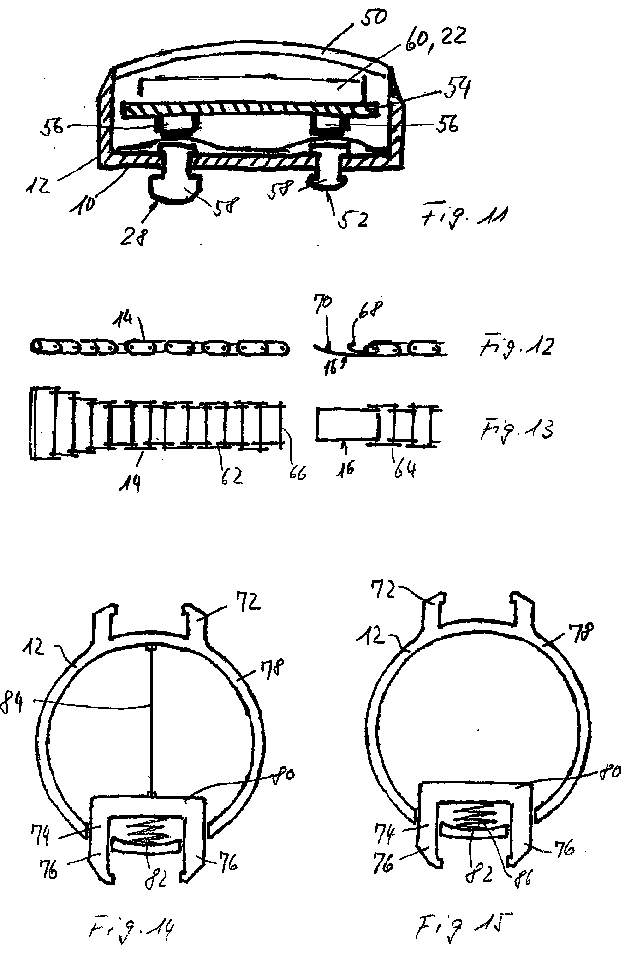

Wie dies der Fig. 11 entnehmbar ist, besteht im gezeigten erfindungsgemäßen Ausführungsbeispiel der Unterschied zwischen den Drucksensoren 28 und dem Anlagedrucksensor 52 darin, dass letzerer weniger als, vorzugsweise etwa die Hälfte wie die Drucksensoren 28 über die freie Oberfläche der Auflageplatte 10 vorsteht.

Zwischen etwa 0.5 mm und etwa 1 mm über die Auflageplatte 10 vorstehende Drucksensoren 28 ergeben, wie dies Versuche gezeigt haben, gute Messresultate und erlauben ein angenehmes Tragen des Geräts.

Im Innern des Gehäuses 12 befindet sich eine Printplatte 54, auf welcher die elektronische Schaltung und die piezoresistiven Sensorelemente 56 der Drucksensoren 28 und des Anlagedrucksensors 52 angeordnet sind. Weiter weisen die Drucksensoren 28 und der Anlagedrucksensor 52 Druckstössel 58 auf, die die Auflageplatte 10 rechtwinklig zu dieser frei bewegbar durchdringen und mit ihrer im Gehäuse 12 angeordneten Stirnseite mit den zugeordneten Sensorelement 56 zusammenwirken. Zu Abdichtungszwecken kann zwischen den Sensorelementen 56 und den Druckstösseln 58 eine Abdichtungsmembrane vorhanden sein, um das Eindringen von Schmutzpartikel oder Wasser zur elektronischen Schaltung zu verhindern. Die Druckstössel 58 sind an ihren äusseren freien Enden vorzugsweise pilzkopfartig abgerundet ausgebildet, um Druckstellen und Verletzungen des Benutzers des Geräts zu verhindern.

Diesbezüglich sind selbstverständlich viele unterschiedliche Lösungen denkbar.

Die Fig. 12 und 13 zeigen eine als Kette ausgebildete Ausführungsform des Bandes 14. Sie weist zwei je als Gelenk-Kette, beispielsweise als Gallsche Gelenk-Kette, ausgebildete Abschnitte 62, 64 auf. Der Abschnitt 62 ist einerends an das Gehäuse 12 angelenkt und ist dazu bestimmt, mit seinen Bolzen 66 in seinem dem Gehäuse abgewandten Endbereich mit einem Haken 68 und einer Sicherungszunge 70 des Verschlusses 16 zusammenzuwirken. Dieser klappenartige, aus der Uhrenindustrie allgemein bekannte Verschluss 16 ist am letzten Haken 68 des Abschnitts 64 schwenkbar befestigt. Der Abschnitt 64 ist mit seinen dem Verschluss 16 abgewandten Ende entweder am Gehäuse 12 oder an einem Bandspannungssensor 18' angelenkt, wie dies im Zusammenhang mit den Fig. 14 und 15 zu beschreiben sein wird.

Die in den Fig. 12 und 13 gezeigte Ausführungsform des Bandes 14 erlaubt in der Art eines Stufenverschlusses das Band 14 in konkreten Schritten enger anzuziehen und zu lösen. Anstelle der gezeigten Ausführung des Verschlusses 16 ist als Stufenverschluss auch ein Verschluss denkbar, wie er von Skischuhen her oder aus der Uhrenindustrie her bekannt ist.

Der Abschnitt 62 bzw. der entsprechende Abschnitt des Bandes 14 wird in bekannter Art und Weise an Befestigungsösen 72 des Gehäuses 12 angelenkt. Um insbesondere bei praktisch unelastischer Ausführungsform des Bandes 14 die Spannung messen zu können, mit welcher das Band 14 angezogen ist, kann der Abschnitt 64 bzw. der entsprechende Abschnitt des Bandes 14 an einen Bandspannungssensor 18' angelenkt sein, wie er in den Fig. 14 und 15 gezeigt ist.

Diese Ausführungsformen des Bandspannungssensors 18' weisen einen C- bzw. U-förmigen Bügel 74 auf. Die beiden zueinander parallelen Schenkel 76 des Bügels 74 bilden Befestigungsösen zur Anlenkung des Abschnitts 64 der Kette bzw. des entsprechenden Abschnitts des Bandes 14. Die beiden Schenkel 76 sind durch die Mantelwand 78 des Gehäuses 12 in ihrer Längsrichtung frei beweglich hindurchgeführt und im Innern des Gehäuses 12 über einen Steg 80 fest miteinander verbunden. Zwischen dem Steg 80 und der Mantelwand 78 wirkt eine Druckfeder 82.

Bei der in der Fig. 14 gezeigten Ausführungsform wirkt zwischen dem Steg 80 und der Mantelwand 78 ein Wegaufnehmer 84. Dieser erzeugt - in Abhängigkeit der Kompression der Druckfeder 82 - ein elektrisches Bandspannungssignal in Abhängigkeit von der Spannung, mit welcher das Band 14 bzw. die Kette angezogen ist.

Bei der in der Fig. 15 gezeigten Ausführungsform ist auf der Druckfeder 82 selber ein DMS-Steifen 86 angeordnet, um ein elektrisches Bandspannungssignal in Abhängigkeit von der Kompression der Druckfeder 82 zu erzeugen.

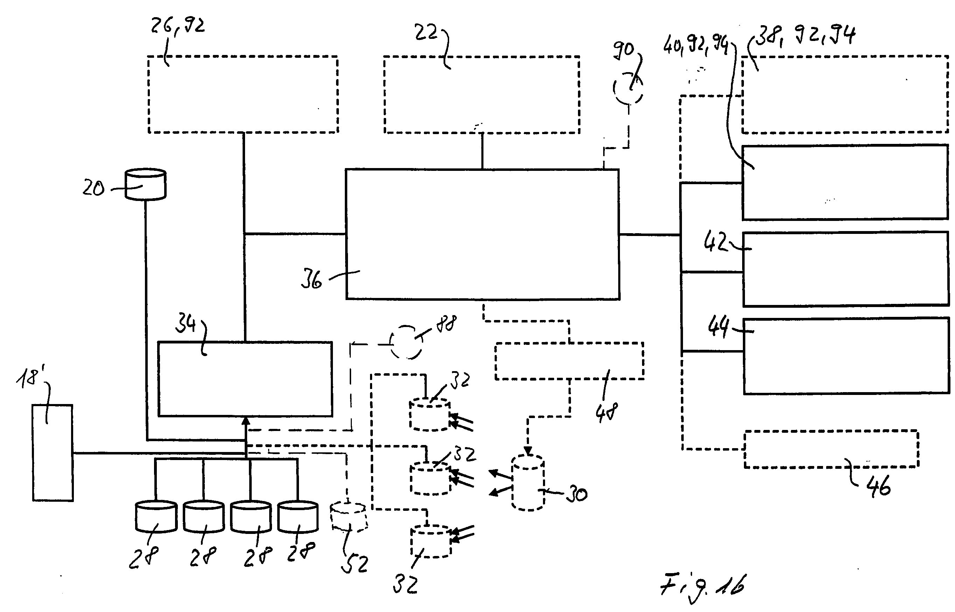

Die in der Fig. 16 gezeigte Ausführungsform der elektronischen Schaltung 60 ist sehr ähnlich jener gemäss Fig. 2.

Die elektrischen Drucksignale der beispielsweise vier Drucksensoren 28, das elektrische Bandspannungssignal des Bandspannungssensors 18' und das elektrische Luftdruck-Outputsignal des Luftdrucksensors 20 werden dem AD-Converter 34 (analog/digital-Wandler) zugeführt, welcher die entsprechenden gewandelten Signale dem Mikroprozessor 36 zuleitet. An dieser Stelle sei vermerkt, dass auf den Luftdrucksensor 20 verzichtet werden kann, wenn die Drucksensoren 28 und gegebenenfalls der Anlagedrucksensor 52 nicht als Absolutdrucksensoren sondern als Relativdrucksensoren ausgebildet sind. Das Luftdruck-Outputsignal wird im Mikroprozessor 36 zur Luftdruckabhängigen Korrektur der Drucksignale bzw. des Anlagedrucksignals verwendet, um entsprechende vom Luftdruck unabhängige Signale für die Weiterverarbeitung zu erhalten. Falls anstelle des Bandspannungssensors 18' oder zusätzlich zu diesem ein Anlagedrucksensor 52 vorhanden ist, wird auch dessen Anlagedrucksignal den AD-Converter 34 und von diesem den Mikroprozessor 36 zugeführt.

An den Mikroprozessor 36 sind weiter, in allgemein bekannter Art und Weise, das USB-Interface 40, der Speicher 42 und die Stromversorgung 44, vorzugsweise mit einer Batterie oder einem Akkumulator, angeschlossen.

Vorzugsweise sind ein Beschleunigungssensor 46 und ein RF-Interface 38 vorhanden, welche ebenfalls an den Mikroprozessor 36 angeschlossen sind.

Weiter ist, falls vorhanden, der Sender des Infrarotsensors 30 über den Verstärker und DA-Wandler 48 mit dem Mikroprozessor 36 verbunden. Die gegebenenfalls vorhandenen, im gezeigten Beispiel drei Detektoren 32 des Infrarotsensors 30 geben ihre Outputsignale an den AD-Converter 34 und über diesen an den Mikroprozessor 36 ab.

Weiter sind auch die Eingabetasten 26 und das Display 22 mit dem Mikroprozessor 36 verbunden.

Der Vollständigkeit halber sei erwähnt, das auch ein Temperatursensor 88 vorhanden sein kann, dessen elektrisches Signal über den AD-Converter 34 dem Mikroprozessor 36 zugeführt wird. Der Temperatursensor 88 kann beispielsweise dazu dienen, die Temperatur des Probanden an der Messstelle und/oder die Temperatur in der elektronischen Schaltung 60 zu messen, um Informationen über die Temperatur des Probanden zu erhalten oder allenfalls Temperaturabhängige Signale zu korrigieren oder das Erreichen und Einhalten der Betriebstemperaturen zu überwachen. Weiter kann an den Mikroprozessor ein optischer oder akustischer Alarmgeber 90 angeschlossen sein. Selbstverständlich ist es denkbar, einen optischen Alarmgeber in das Display 22 zu integrieren.

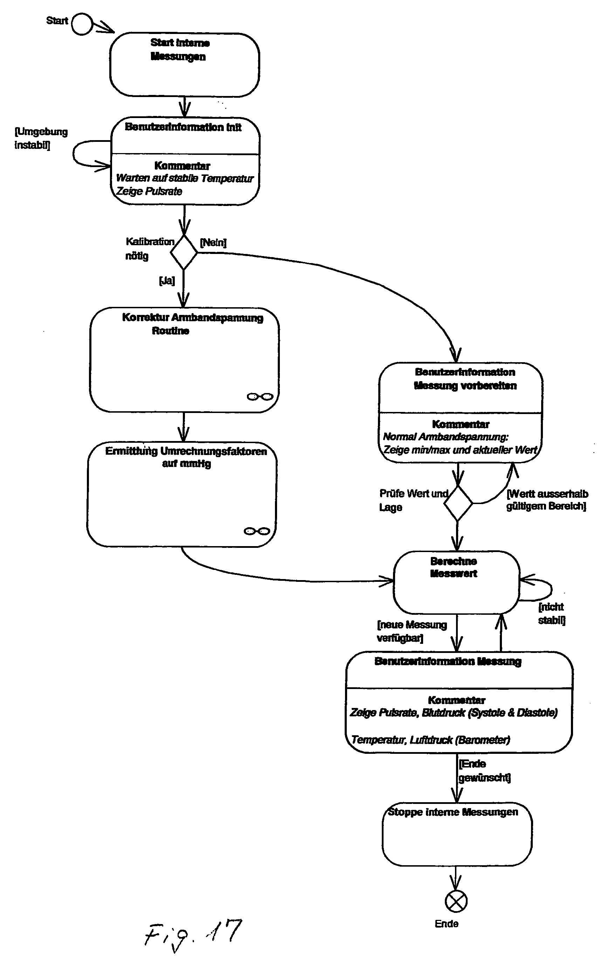

Die elektronische Schaltung besitzt ein Eingabeorgan 92, dieses kann einzelweise oder in Kombination durch die Eingabetasten 26, das RF-Interface 38 und das USB-Interface 40 gebildet sein. Das Eingabeorgan 92 dient der Eingabe von Befehlen und extern ermittelten Werten, worauf im Zusammenhang mit der Beschreibung der Flussdiagramme in Fig. 17, 18 und 20 einzugehen ist. Das USB-Interface 40 dient weiter der Verbindung zu einem externen Computer für die Programmierung des Mikroprozessors 36 und gegebenenfalls zur Übermittlung von mittels des Gerätes erfassten Daten zur weiteren Auswertung an den Computer. Entsprechendes trifft für das RF-Interface 38 zu.

Schlussendlich weist die elektronische Schaltung 60 auch ein Ausgabeorgan 94 auf. Dieses kann einzelweise oder in Kombination durch das Display 22, das RF-Interface 38 und das USB-Interface 40 gebildet sein. Das Display 22 dient der Anzeige der Messergebnisse, von Alarmen, von Zuständen und Anweisungen zur Bedienung. Das RF-Interface 38 und USB-Interface 40 dienen als Ausgabeorgan der Übertragung von ermittelten und gegebenenfalls internen Daten an den externen Computer.

Die Bedienung und Funktionsweise des Geräts sowie die erfindungsspezifische Verarbeitung der Signale im Mikroprozessor 36 geht aus den in den Fig. 17, 18 und 20 gezeigten Flussdiagrammen hervor. Das Flussdiagramm der Fig. 17 gibt einen Überblick über die wichtigsten Schritte nach dem Anlegen des Gerätes an die geeignete Stelle auf der Haut des Körpers, bis nach Beendigung der gewünschten Messungen des Blutdrucks.

Nachdem das Gerät (Fig. 1 oder 8) an einer geeigneten Stelle an einer Extremität angebracht worden ist und das die Extremität umschlingende Band 14 derart angezogen worden ist, dass das Gerät angenehm und verrutschfest an der Stelle sitzt, wird das Gerät, beispielsweise durch Drücken der Eingabetaste 26 "START", eingeschaltet. Dadurch werden interne Messungen, wie Stablitätsmessungen und Funktionskontrollen gestartet.

Sobald diese erfolgreich abgeschlossen sind, geht der Mikroprozessor 36 zu einer die Benutzerinformation Initialisierung betreffende Routine "Benutzerinformation Init" weiter. Dabei überprüft der Mikroprozessor 36 beispielsweise, ob die Umgebung stabil ist, insbesondere ob die mittels des Temperatursensors 88 ermittelte, in diesem Fall interne Temperatur des Gerätes stabil ist. Es wird bereits das Drucksignal des bzw. der Drucksensoren 28 ausgewertet und wird die ermittelte Pulsrate zusätzlich zum Kommentar "Warten auf stabile Temperatur" am Display 22 angezeigt.

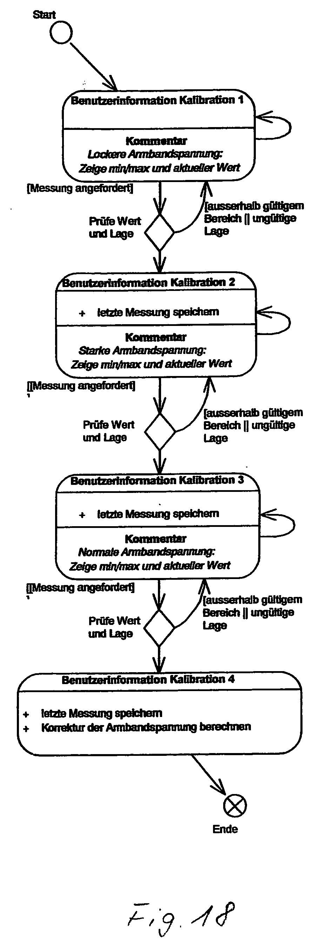

Sobald der Mikroprozessor 36 stabile Bedingungen feststellt, wird entschieden, ob eine Kalibration nötig ist oder nicht. Das Gerät selber entscheidet sich für eine Kalibration beispielsweise wenn es das erste Mal verwendet wird, wenn es seit einer bestimmten längeren Zeit nicht mehr verwendet worden ist, wenn Werte, beispielsweise zu Korrektur- und Untersuchungsfunktionen, nicht vorhanden sind oder als nicht zuverlässig erkannt werden u.s.w. Dieser Entscheid kann jedoch gegebenenfalls auch durch eine bedienende Person oder den Benutzer gefällt werden (falls diese Option vorgesehen ist), wozu auf dem Display 22 eine entsprechende Frage erscheint. Durch Drücken der Eingabetaste 26 "JA" wird eine Kalibration initialisiert, wonach eine "Korrektur Armbandspannung Routine" und dann eine Routine "Ermittlung Umrechnungsfaktoren auf mmHg" durchlaufen werden, wonach die kontinuierliche Messung des Blutdrucks mit der Berechnung der Messwerte im Mikroprozessor 36 startet. Auf die "Korrektur Armbandspannung Routine" und die Routine "Ermittlung Umrechnungsfaktoren auf mmHg" wird im Zusammenhang mit den Fig. 18 - 21 näher eingegangen.

Ist jedoch keine Kalibration nötig, wird dies durch das Drücken der Eingabetaste 26 "NEIN" bestätigt.

Ist keine Kalibration nötig, durchläuft das Gerät die Routine "Benutzerinformation Messung vorbereiten". Dabei erscheint am Display der Hinweis "Normale Armbandspannung:"; dies bedeutet, dass eine normale (mittlere) Armbandspannung vorhanden sein sollte und es wird ein vorbestimmter minimal zulässiger, ein vorbestimmter maximal zulässiger und der aktuelle Wert der Armbandspannung angezeigt. Die Armbandspannung ergibt sich aus dem Bandspannungssignal des Bandspannungssensors 18' oder, falls kein solcher vorhanden ist, aus dem Anlagedrucksignal des Anlagedrucksensors 52. Der Mikroprozessor 36 prüft, ob der Wert in einem gültigen Bereich zwischen dem minimal und maximal zulässigen Wert liegt, und initialisiert, falls dies nicht zutrifft, mittels der Anzeige am Display dass das Band 14 stärker oder weniger stark anzuspannen ist. Dieser Schritt wird wiederholt, bis die gemessene Armbandspannung bzw. der gemessene Anlagedruck im gültigen Bereich liegt. Sobald dies der Fall ist, beginnt die kontinuierliche Messung des Blutdrucks mit der Berechnung der Messwerte.

Die Abfrage der Signale der Sensoren erfolgt in bevorzugterweise mit einer Rate von etwa 40 Hz. Dies ermöglicht eine sehr genaue Auswertung der gemessenen Druck-, Temperatur- und Beschleunigungssignale.

In der Routine "Berechne Messwert" wird neben der Errechnung der Pulsrate, des Blutdrucks (Systole und Diastole), der Temperatur und des Luftdrucks auch laufend überprüft, ob stabile Verhältnisse vorliegen. Dies beispielsweise durch Überprüfung auf Plausibilität und, ob die Messwerte bzw. deren Änderungen innerhalb von vorgegebenen Grenzwerten liegen. Werden nicht stabile Verhältnisse über eine bestimmte Zeit feststellt, dann initialisiert der Mikroprozessor 36 den Alarmgeber 90, um den Benutzer oder die Bedienungsperson darauf aufmerksam zu machen.

Liegen stabile Verhältnisse vor, werden die jeweils errechneten Messwerte, Pulsrate, Blutdruck (Systole und Diastole), Temperatur- und Luftdruck am Display 22 angezeigt, was in der Routine "Benutzerinformation Messung" erfolgt.

Ist das Ende der Messung gewünscht, wird die entsprechende Eingabetaste 26 betätigt. Dadurch werden die internen Messungen im Gerät gestoppt ("Stoppe interne Messungen").

Es ist möglich, berechnete Messwerte und die Korrekturfunktionen im Speicher 42 gespeichert zu halten, wenn anschliessend das Gerät abgeschaltet wird.

Es sei auch erwähnt, dass die Eingaben und die Anzeigen über einen externen Computer erfolgen können, wenn dieser, beispielsweise über das USB-Interface 40, am Gerät angeschlossen ist bzw. bleibt.

Anhand der Fig. 14 wird nun die Ermittlung der Korrekturfunktion für die Armbandspannung mittels der "Korrektur Armbandspannung Routine" beschrieben. Nach dem Start dieser Routine erfolgt der Schritt "Benutzerinformation Kalibration 1", bei welchem das Bandspannungssignal des Bandspannungssensors 18' bzw. das Anlagedrucksignal des Anlagedrucksensors 52 ausgewertet wird. Dazu erscheint am Display 22 der Kommentar "Lockere Armbandspannung" und werden ein vorbestimmter minimal und ein vorbestimmter maximal zulässiger sowie der aktuelle Wert angezeigt. Liegt der aktuelle Wert oberhalb des maximal zulässigen Werts, muss die Bedienperson der der Benutzer das Band 14 mittels des Verschlusses 16 lockern, bis der aktuelle Wert im zulässigen Bereich liegt. Entsprechend muss das Band 14 stärker angezogen werden, wenn der aktuelle Wert unterhalb des minimal zulässigen Werts liegt. Der Mikroprozessor 36 prüft, ob der aktuelle Wert innerhalb des gültigen Bereichs zwischen dem Minimal- und Maximalwert liegt.

Mit Hilfe der Signale des Beschleunigungssensors 46, es handelt sich dabei vorzugswiese um einen 3-Achs-Beschleunigungssensor, stellt der Mikroprozessor 36 sicher, dass alle Kalibrationsmessungen in ruhiger und gleicher Haltung des Messstelle durchgeführt werden.

Liegt der gemessene Wert innerhalb des gültigen Bereichs, geht der Mikroprozessor 36 zum Schritt "Benutzerinformation Kalibration 2". Zu Beginn dieses Schrittes wird die letzte Messung der Armbandspannung bzw. des Anlagedrucks gespeichert, zusammen mit den Drucksignalen der Drucksensoren 28. Anschliessend wird am Display der Kommentar "Starke Armbandspannung!" initialisiert und werden der entsprechende vorbestimmte minimale und vorbestimmte maximale sowie der aktuell gemessene Wert angezeigt. Der Benutzer bzw. eine Bedienungsperson hat das Band 14, vorzugsweise schrittartig, stärker anzuziehen, bis die Überprüfung des Wertes mittels des Mikroprozessors 36 und somit die Anzeige ergibt, dass er innerhalb des gültigen Bereichs zwischen dem maximalen und dem minimalen Wert liegt.

Liegt das Bandspannungssignal bzw. das Anlagedrucksignal innerhalb des gültigen Bereichs, geht der Mikroprozessor 36 zum Schritt "Benutzerinformation Kalibration 3". Zu Beginn dieses Schrittes wird die vorgängige letzte Messung bei starker Bandspannung gespeichert, nämlich das Bandspannungssignal bzw. Anlagedrucksignal und die gleichzeitig gemessenen Drucksignale der Drucksensoren 28.

Dann erscheint am Display der Kommentar "Normale Armbandspannung" und werden entsprechende vorbestimmte maximal und minimal zulässige Werte und der aktuell gemessene Wert angezeigt. Der Benutzer bzw. eine Bedienperson muss mittels des Verschlusses 16 dann die Spannung des Bandes 14 wieder reduzieren, bis der Mikroprozessor 36 feststellt, dass der aktuelle Wert innerhalb eines gültigen Bereiches liegt, was am Display 22 sichtbar gemacht wird.

Hat die Prüfung mittels des Mikroprozessors 36 ergeben, dass der aktuelle Wert innerhalb des zulässigen gültigen Bereichs liegt, erfolgt der Schritt "Benutzerinformation Kalibration 4". Dabei wird die letzte Messung bei normaler Armbandspannung gespeichert, d.h. werden das betreffende Bandspannungssignal bzw. Anlagedrucksignal und die gleichzeitig gemessenen Drucksignale der Drucksensoren 28 gespeichert. Hier sei erwähnt, dass während der Ausführung der "Korrektur Armbandspannung Routine" darauf zu achten ist, dass das Gerät (insbesondere die Drucksensoren 28 und gegebenenfalls der Anlagedrucksensor 52) an Ort und Stelle verbleiben und der Benutzer sich so verhält, dass sein Blutdruck möglichst konstant ist.

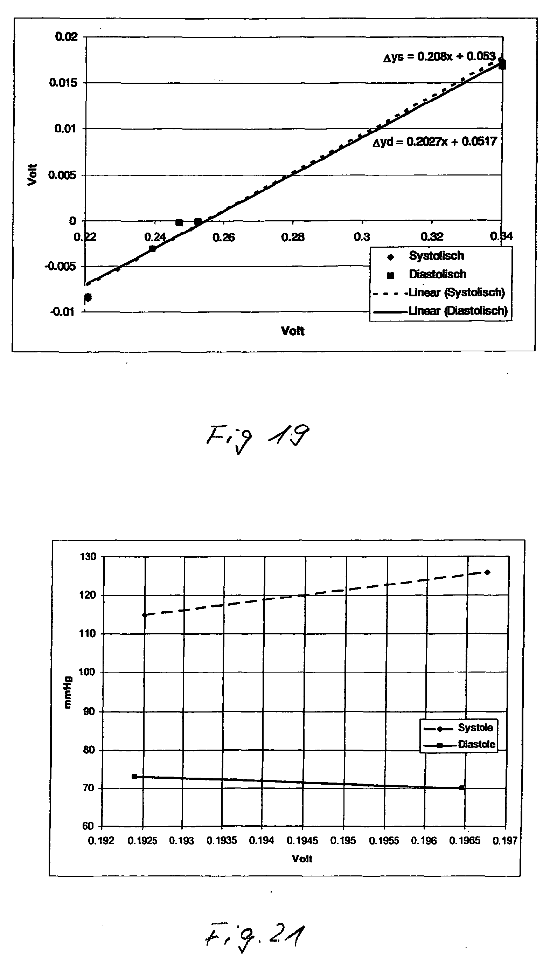

Aus den für die lockere Armbandspannung, die starke Armbandspannung und die normale Armbandspannung gemessenen Werten, wird für die spätere Korrektur der Armbandspannung eine Korrekturfunktion errechnet, wie dies anhand der Fig. 19 im Folgenden beschrieben wird.

In Fig. 19 gibt die Abszisse die gemessene Spannung (in Volt) des Bandspannungssignals bzw. des Anlagedrucksignals an. Die Ordinate betrifft die gemessene Spannung (in Volt) des nullpunktkorrigierten Drucksignals der Drucksensoren 28. Der besseren Übersichtlichkeit halber sind in der Fig. 19 nur die aus dem Drucksignal eines einzigen Drucksensors 28 an einem Testbenutzer ermittelten Werte der Systole (Raute) und Diastole (Quadrat) in Abhängigkeit des Bandspannungssignals bzw. Anlagedrucksignals zu den drei oben beschriebenen Kalibrationsschritten (lockere Armbandspannung, normale Armbandspannung und starke Armbandspannung aufgetragen. Aus diesen Kalibrationsmesswerten lässt sich mittels linearer Regression eine Korrekturfunktion (für jeden Drucksensor 28) ermitteln, welche in der Fig. 19 (für den bestimmten Drucksensor 28) für Diastole mit einer ausgezogenen Geraden und für die Systole mit einer gestrichelten Geraden dargestellt ist.

Für die Diastole wird mittels der linearen Regression eine Korrekturfunktion

$$\Delta yd = kd * x + dd$$

und für die Systole eine Korrekturfunktion

$$\Delta ys = ks * x + ds$$

ermitteln, wobei x die Bandspannung bzw. der Anlagedruck in Millivolt und die Faktoren kd bzw. ks die Steigung und die Faktoren dd bzw. ds den Offset und $\Delta yd$ bzw. $\Delta ys$ den Korrekturwert in Abhängigkeit der Bandspannung bzw. des Anlagedrucks angeben.

Im gezeigten Beispiel wurden für die Systole die Korrekturfunktion $\Delta ys = 0.208x + 0.053$ und für die Diastole die Korrekturfunktion $\Delta yd = 0.2027x + 0.0517$ errechnet.

Während der Routine "Berechne Messwert" wird der jeweils aus dem Drucksignal hervorgehende Wert für die Systole und Diastole in Abhängigkeit des Bandspannungs- bzw. Anlagedrucksignals korrigiert, nach der Formel

$$yd = \text{gemessener Wert der Diastole} - \Delta yd$$

bzw. für die Systole

$$ys = \text{gemessener Wert der Systole} - \Delta ys.$$

Dadurch erfolgt die Kompensation der Änderung der Bandspannung beispielsweise infolge der Änderung des Umfangs der betreffenden Extremität durch Betätigung, Luftdruckänderung oder dergleichen.

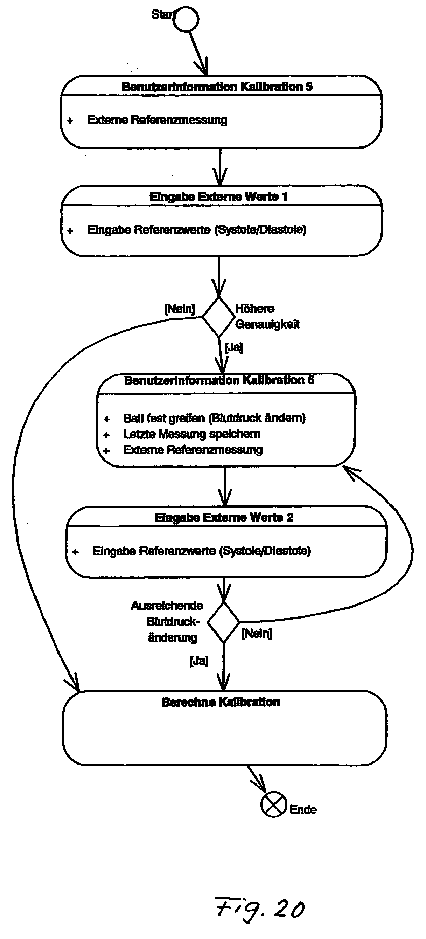

Um aus den über die Korrekturfunktionen für die Armbandspannung korrigierten Werten für die Systole und Diastole die effektiven Blutdruckwerte ermitteln zu können, ist eine weitere Kalibration notwendig, welche unter der Routine "Ermittlung Umrechnungsfaktoren" erfolgt und anhand der Fig. 20 näher erläutert wird. Der Vollständigkeit halber sei erwähnt, dass sowohl für diesen Kalibrationsschritt als auch die Ermittlung der Korrekturfunktion für die Armbandspannung davon ausgegangen wird, dass der Blutdruck des Benutzers des Geräts während der Kalibrationszeit konstant bleibt. Dies kann einerseits dadurch gewährleistet werden, dass der Benutzer die gleiche bequeme Lage beibehält und andererseits der Mikroprozessor eine Plausibilitätsüberprüfung der gemessenen Werte anstellt. Andernfalls sind die betreffenden Kalibrationsschritte zu wiederholen.

Nach dem Start der Routine "Ermittlung Umrechnungsfaktoren auf mmHg" erfolgt über das Display 22 unter dem Schritt "Benutzerinformation Kalibration 5" eine Anzeige "Externe Referenzmessung". Dies erfolgt nachdem, beispielsweise während 5 Minuten nach den Messungen für die Ermittlung der Bandspannungsabhängigkeit, der Blutdruck mittels des Geräts gemessen worden ist und dabei wenigstens annähernd konstante Werte ermittelt worden sind. Es ist dann mit einer anerkannten Messmethode, beispielsweise mittels eines geeichten oszillatorischen Blutdruckmessgeräts Blutdruck des Benutzers zu messen. Ist das Gerät beispielsweise am Unterarm in der Nähe des Handgelenks angebracht worden, so wird in bevorzugter Weise der Blutdruck mittels des oszillatorischen Blutdruckmessgeräts am Oberarm desselben Arms gemessen.

Dann fordert das Gerät im Schritt "Eingabe externer Werte 1" durch eine entsprechend Anzeige am Display 22 auf, die extern gemessenen Referenzwerte für Systole und Diastole einzugeben. Diese Eingabe kann entweder über die Eingabetasten 26 erfolgen oder über einen externen, an das Gerät angeschlossenen Computer. Es ist auch denkbar, dass das externe Blutdruckmessgerät direkt mit dem erfindungsgemässen Gerät kommuniziert und so an dieses die gemessenen Referenzwerte Systole und Diastole übermittelt.

Aus den extern mittels der anerkannten oszillatorischen Blutdruckmessung gemessenen Blutdruckwerten (in mmHg) für die Systole und Diastole einerseits und den mittels der Korrekturfunktion $\Delta yd$ und $\Delta ys$ korrigierten Messwerten des Drucksensors 28 beziehungsweise der Drucksensoren andererseits (in mV) wird eine lineare Kalibrationsfunktion

$$Pd = gd * yd + od$$

für die Diastole und

$$Ps = gs * ys + os$$

für die Systole ermittelt.

Es wird davon ausgegangen, dass im Normalfall (= keine höhere Genauigkeit) eine 1-Punkt Kalibration für den Offset (od und os) ausreicht, während die Verstärkungsfaktoren gd und gs Drucksensor-spezifische feste Faktoren darstellen. Diese Verstärkungsfaktoren sind im gezeigten Ausführungsbeispiel abhängig von der Empfindlichkeit des betreffenden Sensorelements 56 (mV/mmHg) multipliziert mit einem von den Eigenschaften des betreffend Druckststössels abhängigen Korrekturfaktoren; relevant können sein die Länge, die Reibungsverhältnisse, die Elastizität/Steifheit, die Dämpfung usw. Folglich sind die Druckstössel 58 möglichst steif ausgebildet um eine gute Sensibilität zu erzielen. Insbesondere wenn das Gerät das erste Mal benutzt wird, reagiert der Mikroprozessor 36 bei der Entscheidung "Höhere Genauigkeit" mit "Ja" um die Verstärkungsfaktoren gd und gs mittels einer 2-Punkte Kalibration zu ermitteln. Bei weiteren Einsätzen des Geräts kann dann der Benutzer oder die Bedienperson bei diesem Entscheid durch Drücken der entsprechenden Eingabetaste "Ja" oder "Nein" die Art der Kalibration wählen, um die Messgenauigkeit zu erhöhen.

Wird auf "Höhere Genauigkeit gewünscht" = "Ja" entschieden, wird die sogenannte 2-Punkt Kalibration durchgeführt. Dazu wird zusätzlich zur weiter oben beschriebenen externen Referenzmessung eine weitere Messung bei geändertem, vorzugsweise höherem Blutdruck durchgeführt. Dazu kann beispielsweise, wie im Schritt "Benutzerinformation Kalibration 5" angegeben, vom Benutzer ein Ball mit der Hand fest gegriffen werden. Dadurch wird der Blutdruck erhöht und diese Erhöhung wird mittels der Drucksensoren 28 ermittelt. Falls diese Messung vom Mikroprozessor 36 nach vorgegebenen Kriterien (z.B. Stablität, Blutdruckunterschied) als annehmbar beurteilt wird, speichert er die entsprechenden mittels der Korrekturfunktion korrigierten Werte für die Systole und die Diastole und fordert er durch entsprechende Anzeige am Display 22 zu einer weiteren externen Referenzmessung auf. Diese erfolgt, wie weiter oben beschrieben, beispielsweise mittels des externen oszillatorischen Blutdruckmessgeräts.

Unter dem Schritt "Eingabe externe Werte 2" wird durch entsprechende Anzeige am Display 22 zur Eingabe der extern ermittelten Referenzwerte für die Systole und Diastole aufgefordert. Die Eingabe erfolgt auf gleiche Art und Weise wie weiter oben im Zusammenhang mit dem Schritt "Eingabe externer Wert 1" beschrieben.

Der Mikroprozessor 36 überprüft dann, ob eine ausreichende Blutdruckänderung vorliegt oder nicht. Wenn nein, erfolgt wiederum eine neue interne und externe Messung bis eine ausreichende Blutdruckänderung vorgelegen hat. Wenn dies der Fall ist, errechnet der Mikroprozessor 36 aufgrund der mittels der Drucksensoren 28 gemessenen und über die Korrekturfunktion korrigierten Werte für die Systole und Diastole sowie die entsprechenden extern gemessenen Referenzwerte Systole und Diastole die linearen Kalibrationsfunktionen für die Diastole und Systole.

Fig. 21 zeigt ein Beispiel von in dieser Art und Weise ermittelten Kalibrationsfunktionen. Die Abszisse gibt das Drucksignal eines Drucksensors 28 korrigiert über die Korrekturfunktion als Spannung in Volt an. In Richtung der Ordinate sind die betreffenden extern gemessenen Referenzwerte für die Systole und Diastole eingetragen. Die Kalibrationsfunktion für die Diastole ist mit einer ausgezogenen Geraden und jene für die Systole mit einer gestrichelten Geraden dargestellt.

Ist keine "Höhere Genauigkeit" gefordert, errechnet der Mikroprozessor 36 aus den mit der ersten externen Referenzmessung ermittelten Blutdruckwerten und den entsprechenden mit den Drucksensoren ermittelten und mit der Korrekturfunktion korrigierten Werten für die Diastole und Systole den Offset od für die Diastole und os für die Systole, wobei die früher ermittelten und gespeicherten Werte die Verstärkungsfaktoren gq uns gs verwendet werden.

Nach der Berechnung der Kalibrationsfunktionen kann die kontinuierliche Messung und Berechnung der blutdruckwerte starten, wie dies im Zusammenhang mit der Fig. 17 beschrieben worden ist. Während der Messung aktiviert der Mikroprozessor 36 den Alarmgeber 90 im Falle die Blutdruckwerte Diastole oder Systole obere und untere Grenzwerte überschreiten. Der Vollständigkeit halber sei erwähnt, dass die über lange Zeit kontinuierlich ermittelten Blutdruckwerte zusammen mit einer Zeitinformation im Speicher 42 gespeichert werden können und diese Daten dann über eines der Interface 38, 40 an einen externen Computer ausgegeben werden können zur weiteren Auswertung und Archivierung.

Das Gerät kann in der Grössenordnung einer Armbanduhr gebaut werden. Da weiter zur Messung keine Arterie okkludiert oder teilokkludiert werden muss, eignet sich das Gerät auch zu kontinuierlichen Überwachung der Blutdruck- und Pulswerte im Alltagsleben ohne Einschränkungen für den Benutzer.

Aus den Signalen der Drucksensoren 28 kann der Mikroprozessor 36 beispielsweise jenes Signal mit den besten Blutdrucksignalen zur Weiterverarbeitung auswählen. Bei der Ausführungsform mit Drucksensoren 28 und einem Anlagedrucksensor 52 kann der Mikroprozessor 36 weiter, aufgrund der entsprechenden Signale, auch bestimmen, welcher oder welche dieser Sensoren 28, 52 als Anlagedrucksensor 28 eingesetzt wird.

**Patentansprüche**

1. Gerät zum kontinuierlichen Messen des Blutdrucks eines Benutzers zu Überwachungazwecken, mit einem Gehäuse (12) mit einer, einen Gehäuseboden bildenden Auflageplatte (10), mindestens einem Drucksensor (28), der dazu geeignet ist, an einem Ort der externen Oberfläche des Körpers des Benutzers anzuliegen, kontinuierlich den durch den Blutdruck beeinflussten Druck am Ort zu messen und ein entsprechendes elektrisches Drucksignal zu erzeugen, einem Band (14), das dazu geeignet ist, den Körper zu umfassen und den Drucksensor (28) am Ort in sicherer, funktionsfähiger Anlage an der Oberfläche zu halten sowie einer elektronischen Schaltung (60) mit einer Stromversorgung (44), einem Mikroprozessor (36) zur Ermittlung eines diastolischen und eines systolischen Blutdruckwerts und einem Ausgabeorgan (94) zur Anzeige beziehungsweise Ausgabe der Blutdruckwerte, einem Anlagedrucksensor (52), der geeignet ist, an der externen Oberfläche des Körpers des Benutzers anzuliegen, kontinuierlich den Anlagedruck zu messen und ein entsprechendes elektrisches Anlagedrucksignale zu erzeugen, wobei der Mikroprozessor (36) unter Berücksichtigung des Anlagedrucksignals den diastolischen und den systolischen Blutdruckwert ermittelt, **gekennzeichnet dadurch, dass** der Drucksensor (28) gegenüber der Auflageplatte (10) vorsteht und dass der Anlagedrucksensor (52) ebenfalls über der Auflageplatte (10) vorsteht, jedoch weniger als der Drucksensor (28).

2. Gerät nach Anspruch 1, **dadurch gekennzeichnet, dass** der Drucksensor (28) um maximale 1 mm gegenüber der Auflageplatte (10) vorsteht.

3. Gerät nach Anspruch 1 oder 2, **dadurch gekennzeichnet, dass** der Anlagedrucksensor (52) um etwa die Hälfte des Drucksensors (28) über die Auflageplatte (10) vorsteht.

4. Gerät nach einem der Ansprüche 1 bis 3, **dadurch gekennzeichnet, dass** zusätzlich zum Anlagedrucksensor (52) ein Bandspannungssensor (18') vorhanden ist, der geeignet ist, die Zugspannung des Bandes (14) kontinuierlich zu messen und ein entsprechendes elektrisches Bandspannungssignal zu erzeugen, wobei der Mikroprozessor (36) unter Berücksichtigung des Bandspannungssignals den diastolischen und den systolischen Blutdruckwert ermittelt.

5. Gerät nach einem der Ansprüche 1 bis 4, **dadurch gekennzeichnet, dass** der Mikroprozessor (36) dazu geeignet ist, bei, vorzugsweise stufenartig, unterschiedlich angezogenem Band (14), in Abhängigkeit vom entsprechenden Anlagedrucksignal beziehungsweise sandspannungssignal und dem Drucksignal eine Korrekturfunktion, vorzugsweise durch lineare Regression, zu ermitteln, um - beim kontinuierlichen Messen - das Drucksignal in Abhängigkeit vom Analagedrucksignal beziehungsweise Bandspannungssignal zu korrigieren.

6. Gerät nach einem der Ansprüche 1 bis 5, **dadurch gekennzeichnet, dass** das Band (14) mit einem Verschluss (16), vorzugsweise einem Stufenverschluss, versehen ist, um das Band (14) stärker und schwächer anziehen zu können.

7. Gerät nach einem der Ansprüche 1 bis 6, **gekennzeichnet durch** ein Eingabeorgan (92) zur Eingabe mindestens eines extern gemessenen diastolischen und systolischen Effektivblutdruckwerts, wobei der Mikroprozessor (36) geeignet ist, aus diesen Effektivblutdruckwerten und dem, gegebenenfalls mittels der Korrekturfunktion korrigierten, Drucksignal eine Kalibrationsfunktion zu ermitteln, um - beim kontinuierlichen Messen - die effektiven Blutdruckwerte zu ermitteln.

8. Gerät nach einem der Ansprüche 1 bis 7, **gekennzeichnet durch** einen Luftrucksensor (20) dessen elektrisches Luftdruck-Outputsignal dem Mikroprozessor (36), vorzugsweise zur Korrektur des Drucksignals und gegebenenfalls des Anlagedrucksignals, zugeführt werden.

9. Gerät nach Anspruch 8, **dadurch gekennzeichnet, dass** der Drucksensor (28) als Absolutdrucksensor ausgebildet ist und, falls vorhanden, der Anlagedrucksensor (52) vorzugsweise ebenfalls als Absolutdrucksensor ausgebildet ist.

10. Gerät nach einem der Ansprüche 1 bis 9, **gekennzeichnet durch** einen Beschleunigungssensor (46), dessen elektrisches Beschleunigung-Outputsignal dem Mikroprozessor (36) zur Auswertung zugeführt wird.

11. Gerät nach einem der Ansprüche 1 bis 10, **gekennzeichnet durch** einen Temperatursensor (88), dessen elektrisches Temperatur-Outputsignal dem Mikroprozessor (36) zur Auswertung zugeführt wird.

12. Gerät nach einem der Ansprüche 1 bis 11, **gekennzeichnet durch** einen Infrarotsender (30) mit zugeordnetem Detektor (32), dessen elektrisches Detektor-Outputsignal dem Mikroprozessor (36) zur Auswertung zugeführt wird.

13. Gerät nach einem der Ansprüche 1 bis 12, **dadurch gekennzeichnet, dass** die elektronische Schaltung (60) einen akustischen oder optischen Alarmgeber (24) aufweist, welcher bei über- oder unterschreiten von vorgebbaren Alarmgrenzwerten für den diastolischen und systolischen Blutdruck vom Mikroprozessor (36), zur Auslösung eines Alarms, ansteuerbar ist.

## Claims

1. Apparatus for continuously measuring the blood pressure of a user for monitoring purposes, with a housing (12) with a bearing plate (10), which forms a housing base, at least one pressure sensor (28) suitable for resting against a site on the external surface of the body of the user, continuously measuring the pressure at the site influenced by the blood pressure and generating a corresponding electrical pressure signal, a band (14) suitable for encompassing the body and for holding the pressure sensor (28) against the surface at the site with safe, functional contact, as well as an electronic circuit (60) with a current supply (44), a microprocessor (36) for establishing a diastolic and a systolic blood-pressure value, and an output element (94) for displaying and outputting, respectively, the blood-pressure values, a contact-pressure sensor (52) which is suitable for resting against the external surface of the body of the user, continuously measuring the contact pressure and generating a corresponding electrical contact-pressure signal, wherein the microprocessor (36) taking into account the contact-pressure signal establishes the diastolic and the systolic blood-pressure value, **characterized in that** the pressure sensor (28) protrudes with respect to the bearing plate (10) and **in that** the contact-pressure sensor (52) also protrudes with respect to the bearing plate (10), however less than the pressure sensor (28).

2. Apparatus as claimed in claim 1, **characterized in that** the pressure sensor (28) protrudes with respect to the bearing plate (10) by at most 1 mm.

3. Apparatus as claimed in claim 1 or 2, **characterized in that** the contact-pressure sensor (52) protrudes over the bearing plate (10) by about the half of the pressure sensor (28).

4. Apparatus as claimed in any one of claims 1 to 3, **characterized in that** in addition to the contact-pressure sensor (52) a band-tension sensor (18') is present, which is suitable for continuously measuring the tensile stress in the band (14) and for generating a corresponding electrical band-tension signal, wherein the microprocessor (36) taking into account the band-tension signal establishes the diastolic and the systolic blood-pressure value.

5. Apparatus as claimed in any one of claims 1 to 4, **characterized in that** the microprocessor (36) is suitable for establishing a correction function, preferably by linear regression, as a function of the corresponding contact-pressure signal and band-tension signal, respectively, and the pressure signal in the case of the band (14) being tightened to a different extent, preferably in a step-like fashion, in order to correct the pressure signal - during the continuous measurement - as a function of the contact-pressure signal and band-tension signal, respectively.

6. Apparatus as claimed in any one of claims 1 to 5, **characterized in that** the band (14) is provided with a catch (16), preferably a stepped catch, in order to be able to tighten the band (14) to a greater and lesser extent.

7. Apparatus as claimed in any one of claims 1 to 6, **characterized by** an input element (92) for entering at least one externally measured diastolic and systolic effective blood-pressure value, wherein the microprocessor (36) is suitable for establishing a calibration function from these effective blood-pressure values and the pressure signal, which was corrected by the correction function where applicable, in order to establish the effective blood-pressure values - during the continuous measurement -.

8. Apparatus as claimed in one of claims 1 to 7, **characterized by** an air-pressure sensor (20), the electrical air-pressure output signal of which is fed to the microprocessor (36), preferably for correcting the pressure signal and, where applicable, for correcting the contact-pressure signal.

9. Apparatus as claimed in claim 8, **characterized in that** the pressure sensor (28) is embodied as an absolute-pressure sensor and, if present, the contact-pressure sensor (52) is preferably likewise embodied as an absolute-pressure sensor.

10. Apparatus as claimed in one of claims 1 to 9, **characterized by** an accelerometer (46), the electrical acceleration output signal of which is fed to the microprocessor (36) for evaluation.

11. Apparatus as claimed in one of claims 1 to 10, **characterized by** a temperature sensor (88), the electrical temperature output signal of which is fed to the microprocessor (36) for evaluation.

12. Apparatus as claimed in one of claims 1 to 11, **characterized by** an infrared transmitter (30) with an associated detector (32), the electrical detector output signal of which is fed to the microprocessor (36) for evaluation.

13. Apparatus as claimed in one of claims 1 to 12, **characterized in that** the electronic circuit (60) has an acoustic or optical alarm transmitter (24), which can be actuated by the microprocessor (36) in order to trigger an alarm if prescribable alarm thresholds for the diastolic and systolic blood pressure are exceeded or undershot.

**Revendications**

1. Dispositif pour la mesure continue de la tension artérielle d'un utilisateur à des fins de contrôle, avec un boîtier (12) ayant une plaque de support (10) qui forme une base de boîtier, au moins un senseur de pression (28) adapté pour être ajusté à un endroit à la surface extérieure du corps de l'utilisateur, mesurant continuellement la pression influencée par la tension artérielle à l'endroit et générant un signal électrique de pression correspondant, une bande (14) adaptée pour entourer le corps et pour tenir le senseur de pression (28) contre la surface à l'endroit avec un contact sûr, fonctionnel, ainsi qu'un circuit électronique (60) avec une alimentation en courant (44), un microprocesseur (36) pour établir des valeurs de tension artérielle diastolique et systolique, et un élément de sortie (94) pour afficher ou sortir respectivement les valeurs de tension artérielle, un senseur de pression de contact (52) qui est adapté pour être ajusté à la surface extérieure du corps de l'utilisateur, mesurant continuellement la pression de contact et générant un signal électrique de pression de contact correspondant, dans lequel le microprocesseur (36) considérant le signal de pression de contact établit les valeurs de tension artérielle diastolique et systolique, **caractérisé en ce que** le senseur de pression (28) dépasse par rapport à la plaque de support (10) et **en ce que** le senseur de pression de contact (52) dépasse aussi par rapport à la plaque de support (10), toutefois moins que le senseur de pression (28).

2. Dispositif selon la revendication 1, **caractérisé en ce que** le senseur de pression (28) dépasse par rapport à la plaque de support (10) d'au maximum 1 mm.

3. Dispositif selon la revendication 1 ou 2, **caractérisé en ce que** le senseur de pression de contact (52) dépasse de la plaque de support (10) d'environ la moitié du senseur de pression (28).

4. Dispositif selon l'une quelconque des revendications 1 à 3, **caractérisé en ce qu'**en plus du senseur de pression de contact (52) un senseur de tension de bande (18') est présent, qui est adapté pour mesurer continuellement la tension dans la bande (14) et pour générer un signal électrique de tension de bande correspondant, dans lequel le microprocesseur (36) considérant le signal de tension de bande établit les valeurs de tension artérielle diastolique et systolique.

5. Dispositif selon l'une quelconque des revendications 1 à 4, **caractérisé en ce que** le microprocesseur (36) est adapté pour établir une fonction de correction, de préférence par régression linéaire, en fonction du signal de pression de contact ou du signal de tension de bande correspondant respectivement et du signal de pression, lorsque la bande (14) est tendue de façon différente, préférablement par palier, pour corriger le signal pression - pendant la mesure continue - en fonction du signal de pression de contact ou du signal de tension de bande respectivement.

6. Dispositif selon l'une quelconque des revendications 1 à 5, **caractérisé en ce que** la bande (14) est munie d'une fermeture (16), préférablement d'une fermeture par palier, pour pouvoir tendre la bande (14) plus fort et moins fort.

7. Dispositif selon l'une quelconque des revendications 1 à 6, **caractérisé par** un élément d'entrée (92) pour entrer au moins une valeur effective de tension artérielle diastolique et une valeur effective de tension artérielle systolique mesurées extérieurement, dans lequel le microprocesseur (36) est adapté pour établir une fonction de calibration à partir de ces valeurs effectives de tension artérielle et du signal de pression, le cas échéant corrigé par la fonction de correction, pour établir les valeurs effectives de tension artérielle - pendant la mesure continue -.

8. Dispositif selon l'une des revendications 1 à 7, **caractérisé par** un senseur de pression de l'air (20), dont le signal électrique de sortie de pression de l'air est amené au microprocesseur (36), préférablement pour corriger le signal de pression et, le cas échéant, pour corriger le signal de pression de contact.

9. Dispositif selon la revendication 8, **caractérisé en ce que** le senseur de pression (28) est conçu comme un senseur de pression absolue et, si présent, le senseur de pression de contact (52) est préférablement aussi conçu comme un senseur de pression absolue.

10. Dispositif selon l'une des revendications 1 à 9, **caractérisé par** un accéléromètre (46), dont le signal électrique de sortie d'accélération est amené au microprocesseur (36) pour évaluation.

11. Dispositif selon l'une des revendications 1 à 10, **caractérisé par** un senseur de température (88), dont le signal électrique de sortie de température est amené au microprocesseur (36) pour évaluation.

12. Dispositif selon l'une des revendications 1 à 11, **caractérisé par** un émetteur à infrarouge (30) avec un détecteur associé (32), dont le signal électrique de sortie du détecteur est amené au microprocesseur (36) pour évaluation.

13. Dispositif selon l'une des revendications 1 à 12, **caractérisé en ce que** le circuit électronique (60) présente un émetteur d'alarme acoustique ou optique (24), qui est commandable par le microprocesseur (36) pour déclencher une alarme si des seuils d'alarme prédéfinissables pour les tensions artérielles diastolique et systolique sont dépassés ou ne sont pas atteints.

**Fig. 1**

**Fig. 2**

# Fig. 3

| Zeit (s) | Beschl. X-Achse (g) | Beschl. y-Achse (g) | Beschl. Z-Achse (g) | Haut-temp. (°C) | Ref. Temp. (V) | Temp. Druck-sensor1 (V) | Druck-sensor 1 (V) | Druck-sensor 2 (V) | Druck-sensor 3 (V) | Druck-sensor 4 (V) | Ref. sensor (V) |
|---|---|---|---|---|---|---|---|---|---|---|---|
| 0.000 | 0.21 | 1.01 | 0.03 | 31.9 | 2.134 | 2.134 | 0.30471 | 0.31964 | 0.33318 | 0.38031 | 0.26496 |
| 0.017 | 0.21 | 0.98 | 0.05 | 31.9 | 2.134 | 2.134 | 0.30485 | 0.31981 | 0.33327 | 0.38062 | 0.26491 |
| 0.033 | 0.2 | 1 | 0.06 | 31.9 | 2.134 | 2.135 | 0.30473 | 0.31982 | 0.33327 | 0.38097 | 0.26499 |
| 0.050 | 0.21 | 0.99 | 0.03 | 31.9 | 2.134 | 2.134 | 0.30483 | 0.31995 | 0.33327 | 0.38064 | 0.26493 |
| 0.067 | 0.21 | 0.99 | 0.04 | 32 | 2.134 | 2.135 | 0.30496 | 0.31981 | 0.33319 | 0.38057 | 0.26496 |

EP 2 337 493 B1

**Fig. 4**

## Fig. 5

Systole

y = 0.2749x + 3.9804
R² = 0.5801

## Fig. 6

Diastole

y = 0.2738x + 6.0041
R² = 0.3829

## Fig. 7

Vergleich

Diastolische
Werte

Drucksignal [10mV]

Referenz [mmHg]

◆ diastolisch

◆ systolisch

— —Linear
(diastolisch)

—— Linear
(systolisch

Fig. 8

Fig. 9

Fig. 10

Fig. 11

Fig. 12

Fig. 13

Fig. 14

Fig. 15

Fig. 16

Start ◯ ↗

**Start interne Messungen**

↓

**Benutzerinformation init**
_____
**Kommentar**
*Warten auf stabile Temperatur*
*Zeige Pulsrate*

[Umgebung instabil] ↰

↓

Kalibration nötig ◇ [Nein]

[Ja] ↓

**Korrektur Armbandspannung Routine**

∞

↓

**Ermittlung Umrechnungsfaktoren auf mmHg**

∞

**Benutzerinformation Messung vorbereiten**
_____
**Kommentar**
*Normal Armbandspannung:*
*Zeige min/max und aktueller Wert*

Prüfe Wert und Lage ◇ [Wert ausserhalb gültigem Bereich]

↓

**Berechne Messwert** ↻ [nicht stabil]

[neue Messung verfügbar] ↓

**Benutzerinformation Messung**
_____
**Kommentar**
*Zeige Pulsrate, Blutdruck (Systole & Diastole)*

*Temperatur, Luftdruck (Barometer)*

[Ende gewünscht] ↓

**Stoppe interne Messungen**

↓

⊗

Ende

*Fig. 17*

24

Start

Benutzerinformation Kalibration 1

Kommentar
*Lockere Armbandspannung:*
*Zeige min/max und aktueller Wert*

[Messung angefordert]

Prüfe Wert
und Lage

[ausserhalb gültigem
Bereich || ungültige
Lage

Benutzerinformation Kalibration 2

\+   letzte Messung speichern

Kommentar
*Starke Armbandspannung:*
*Zeige min/max und aktueller Wert*

[[Messung angefordert]

Prüfe Wert
und Lage

[ausserhalb gültigem
Bereich || ungültige
Lage

Benutzerinformation Kalibration 3

\+   letzte Messung speichern

Kommentar
*Normale Armbandspannung:*
*Zeige min/max und aktueller Wert*

[[Messung angefordert]

Prüfe Wert
und Lage

[ausserhalb gültigem
Bereich || ungültige
Lage

Benutzerinformation Kalibration 4

\+   letzte Messung speichern
\+   Korrektur der Armbandspannung berechnen

Ende

Fig. 18

Fig 19

Fig. 21

Start ○

**Benutzerinformation Kalibration 5**

+ Externe Referenzmessung

**Eingabe Externe Werte 1**

+ Eingabe Referenzwerte (Systole/Diastole)

[Nein] ◇ Höhere
Genauigkeit

[Ja]

**Benutzerinformation Kalibration 6**

+ Ball fest greifen (Blutdruck ändern)
+ Letzte Messung speichern
+ Externe Referenzmessung

**Eingabe Externe Werte 2**

+ Eingabe Referenzwerte (Systole/Diastole)

Ausreichende
Blutdruckänderung ◇ [Nein]

[Ja]

**Berechne Kalibration**

⊗ Ende

*Fig. 20*

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1341436 A
- WO 9703606 A